# EUROPEAN PATENT APPLICATION

(11) **EP 4 356 845 A1**
(43) Date of publication of application: **24.04.2024**
(21) Application number: 22841155.9
(22) Date of filing: 23.06.2022
(51) Int. Cl.: A61B 17/00, A61B 34/30

(54) **INSTRUMENT DRIVE TRANSMISSION MECHANISM AND ASSEMBLY MECHANISM OF SURGICAL ROBOT**

(30) Priority: 14.07.2021 CN 202110797198
(71) Applicant: Cornerstone Technology (Shenzhen) Limited, Shenzhen, Guandong 518066 (CN)
(72) Inventor: YANG, Qiusheng, Shenzhen, Guangdong 518066 (CN); ZHANG, Jianwei, Shenzhen, Guangdong 518066 (CN)
(74) Representative: Balder IP Law, S.L.
(86) International application number: PCT/CN2022/100859
(87) International publication number: WO 2023/284515

(57) **Abstract**

An instrument driving and transmission mechanism for a surgical robot and an instrument driving and assembly mechanism for the surgical robot are provided. The instrument driving and transmission mechanism includes an instrument drive (100), a sterile adapter (200), and a surgical instrument (300). Adapter transmission members (230) of the sterile adapter (200) and drive transmission members (130) of the instrument drive (100) mesh with each other by means of several teeth (131, 233) that are evenly spaced on surfaces of the adapter transmission members (230) and the drive transmission members (130), and the adapter transmission members (230) of the sterile adapter (200) cooperate with instrument transmission members (311) of the surgical instrument (300) by means of transmission bosses (2321, 2321a, 2321b).

## Description

### CROSS REFERENCE TO RELATED APPLICATIONS

This application claims priority to Chinese Patent Application No. CN202110797198.0, entitled "Instrument driving and transmission mechanism and assembly mechanism for surgical robot," filed on July 14, 2021, which is hereby incorporated by reference in its entirety.

### TECHNICAL FIELD

The various embodiments described in this document relate in general to the technical field of medical instruments, and more specifically to an instrument driving and transmission mechanism and assembly mechanism for a surgical robot.

### BACKGROUND

Surgical robots have a large number of applications in clinical surgery as they help surgeons achieve precise positioning for surgery, have advantages such as reducing patient's wounds and thereby shortening post-operative recovery time, and have a stable operating platform capable of addressing situations such as tremors of surgeons.

A surgical instrument in the surgical robot typically has an end effector in the form of a surgical tool, such as forceps, scissors, clamps, etc., at one end of an elongated tube. In general, wires or ropes are employed to manipulate the end effector to pitch, yaw and grip.

A surgeon controls, at a console side, the instrument connected to a drive at a surgical side. In order to meet demands for different surgical instruments to be used during surgery, surgical instruments and the instrument drive are typically designed to be detachable for changing different surgical instruments during the surgery, and the surgical instruments are typically sterilized independently.

The instrument drive is typically designed to be non-sterilizable, and in order to ensure sterility during the surgery, a sterile adapter needs to be added between the instrument drive and the instrument during the surgery to isolate the non-sterilizable instrument drive and the sterilizable instrument during the surgery.

A back end of the surgical instrument is connected to an upper surface of the sterile adapter, the instrument drive is connected to a lower surface of the sterile adapter, and the instrument drive provides a driving force to the end effector of the surgical instrument through the sterile adapter for achieving pitch, yaw and grip of the end effector.

The lower surface of the sterile adapter is connected to an upper surface of the instrument drive, which are stable and undetached after connection, and meanwhile, the sterile adapter is also able to be easily and quickly unlocked and detached from the instrument drive when needed. The back end of the surgical instrument is connected to the upper surface of the sterile adapter, which are stable and undetached after connection, and meanwhile, the surgical instrument is also able to be easily and quickly unlocked and detached from the sterile adapter when needed.

At present, connection between a transmission portion of the instrument drive and a transmission portion of the sterile adapter is achieved as follows. The instrument drive is provided with two symmetrically arranged engaging members on a transmission disc of the instrument drive, and the sterile adapter defines two recesses, for the two engaging members, on a transmission member of the sterile adapter. During installation, it is necessary to connect a housing of the sterile adapter with a housing of the instrument drive first, and then identify whether the sterile adapter is in place through a sensor. Thereafter, the transmission disc of the instrument drive is driven to rotate by a motor of the instrument drive to perform angle adjustment multiple times, so as to achieve engagement between the two engaging members of the transmission disc of the instrument drive and the two recesses of the transmission member of the sterile adapter. However, there are some problems existed in this manner. For example, when a surface of the housing of the sterile adapter and a surface of the housing of the instrument drive are connected, and connection between the transmission member of the sterile adapter and the transmission disc of the instrument drive is not successful, since a diameter of a mounting hole for the transmission member in the sterile adapter is larger than a diameter of the transmission member of the sterile adapter, the transmission member of the sterile adapter may be slightly tilted, and an axle center of the transmission member of the sterile adapter may not be aligned with a rotating axis of the transmission disc of the instrument drive. Therefore, a respective recess of the sterile adapter may not be very accurate on a rotating curve of a respective engaging member of the two engaging members. During connecting of the transmission disc of the instrument drive with the transmission member of the sterile adapter, the motor of the instrument drive needs to drive the transmission disc of the instrument drive to rotate several turns to perform blind connecting. After the transmission disc of the instrument drive is connected with the transmission member of the sterile adapter, there is a need to perform rotations multiple times to determine if the connection is successful. Moreover, after the transmission disc of the instrument drive is connected with the transmission member of the sterile adapter through engagement of the two engaging members and the recesses, merely the two engaging members are subjected to torsion, which can easily cause excessive wear and tear.

### SUMMARY

A series of simplified concepts have been introduced in this section, which will be further elaborated in the detailed description. The section of the disclosure does not mean attempting to limit key features and essential technical features of the claimed technical solution, nor does it mean attempting to determine the scope of protection of the claimed technical solution.

Embodiments of the disclosure provide an instrument driving and transmission mechanism for a surgical robot. The instrument driving and transmission mechanism includes an instrument drive, a sterile adapter, and a surgical instrument. The instrument drive includes a drive transmission member exposed at an upper surface of the instrument drive. The sterile adapter has a lower surface matching the upper surface of the instrument drive, the sterile adapter includes an adapter transmission member penetrating a body of the sterile adapter, and the adapter transmission member has a lower end cooperated with the drive transmission member. The surgical instrument has a back end matching an upper surface of the sterile adapter, the surgical instrument includes an instrument transmission member exposed at the back end of the surgical instrument, and the instrument transmission member is cooperated with an upper end of the adapter transmission member. The adapter transmission member has a surface on which a plurality of teeth are provided and evenly spaced, the drive transmission member has a surface on which a plurality of teeth are provided and evenly spaced, the plurality of teeth of the adapter transmission member are meshed with by the plurality of teeth the drive transmission member, and the adapter transmission member and the instrument transmission member are cooperated with each other by means of transmission bosses.

In the instrument driving and transmission mechanism of the surgical robot, the adapter transmission member and drive transmission member are meshed with each other by the plurality of teeth evenly spaced on the surface of the adapter transmission member. During installation, the adapter transmission member and the drive transmission member can be fitted at any angle, allowing for fast and precise connection. In addition, the engaging of the multiple teeth provides a stronger torque transfer and greater durability than fitting of one or two transmission bosses.

Embodiments of the disclosure provide an instrument driving and assembly mechanism for a surgical robot, which includes an instrument drive, a sterile adapter, and a surgical instrument box. The instrument drive has an assembly surface connected with the sterile adapter. The sterile adapter has a lower surface matching the assembly surface of the instrument drive. The surgical instrument box has a lower surface matching an upper surface of the sterile adapter. The lower surface of the sterile adapter is connected with the assembly surface of the instrument drive through hook-shaped fitting surfaces and operable engagement assemblies, and the upper surface of the sterile adapter is connected with the lower surface of the surgical instrument through at least two operable engagement assemblies spaced apart from each other.

In the instrument driving and assembly mechanism of the surgical robot, there are multiple connection manners between the instrument drive and the sterile adapter, and there are multiple connection manners between the sterile adapter and the surgical instrument box. The multiple connection manners enable the mechanism to be structurally stronger and to reduce vibration when in use. In addition, connection between the instrument drive and the sterile adapter have a certain degree of rigidity, so that the connection between the instrument drive and the sterile adapter is less prone to structural movement or deformation.

### BRIEF DESCRIPTION OF THE DRAWINGS

The following drawings of this disclosure are herein used as a part of this disclosure for understanding the disclosure. Embodiments of the present disclosure and their description are shown in the accompanying drawings to explain the principles of the present disclosure.
FIG. 1 is a schematic structural view of an instrument drive, a sterile adapter, and a surgical instrument that are assembled according to embodiments of the present disclosure.
FIG. 2 is a schematic view of the instrument drive, the sterile adapter, and the surgical instrument that are not disassembled according to embodiments of the present disclosure.
FIG. 3 is a top view of an instrument drive according to embodiments of the present disclosure.
FIG. 4 is a schematic view of an internal structure of the instrument drive according to embodiments of the present disclosure.

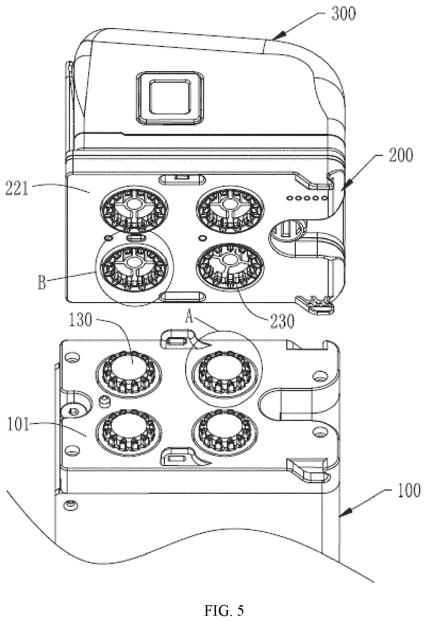

FIG. 5 is a schematic view of a connection structure between the instrument drive and the sterile adapter according to embodiments of the present disclosure.
FIG. 6 is a schematic structural view of a transmission member of the instrument drive according to embodiments of the present disclosure.
FIG. 7 is a schematic exploded view of the sterile adapter according to embodiments of the present disclosure.
FIG. 8 is a schematic structural view of a lower end portion of a transmission member of the sterile adapter according to embodiments of the present disclosure.
FIG. 9 is a schematic exploded view of a transmission connection structure between the sterile adapter and the surgical instrument according to embodiments of the present disclosure.
FIG. 10 is a schematic structural view of the instrument drive and the sterile adapter that are in an assembly state according to embodiments of the present disclosure.
FIG. 11 is a schematic view of a connection structure between the instrument drive and the sterile adapter according to embodiments of the present disclosure.
FIG. 12 is a schematic exploded view of a connection structure between the instrument drive and the sterile adapter according to embodiments of the present disclosure.
FIG. 13 is a schematic exploded view of a connection structure between the instrument drive and the sterile adapter according to other embodiments of the present disclosure.
FIG. 14 is a partial structural schematic view of a connection structure between the instrument drive and the sterile adapter according to embodiments of the present disclosure.
FIG. 15 is an enlarged detail view of a first fixing engaging member of an instrument drive according to embodiments of the present disclosure.
FIG. 16 is a schematic exploded view of a connection structure between the sterile adapter and the instrument drive according to embodiments of the present disclosure.
FIG. 17 is a schematic exploded view of a second connection manner between the sterile adapter and the instrument drive according to embodiments of the present disclosure.
FIG. 18 is a schematic exploded view of a surgical instrument box according to embodiments of the present disclosure.
FIG. 19 is a schematic structural view of a guide backboard of a sterile adapter according to embodiments of the present disclosure.
FIG. 20 is a schematic view of a back structure of a surgical instrument box according to embodiments of the present disclosure.
FIG. 21 is an enlarged detail view of a second fixing engaging member of a sterile adapter according to embodiments of the present disclosure.
FIG. 22 is an enlarged detail view of a second fixing engaging member of a sterile adapter according to other embodiments of the present disclosure.

### DETAILED DESCRIPTION OF THE EMBODIMENTS

In the following description, numerous specific details are given to provide a more thorough understanding of the disclosure. However, it will be apparent to those skilled in the art that embodiments of the present disclosure may be practiced without one or more of these details. In other examples, some technical features well known in the art are not described in order to avoid confusion with embodiments of the present disclosure.

In order to thoroughly understand the embodiments of the present disclosure, detailed structures will be set forth in the following description. Apparently, the implementation of the embodiments of the present disclosure is not limited to the particular details familiar to those skilled in the art. Ordinal numerals such as "first" and "second" referenced in the disclosure are merely identifiers and do not have any specific meaning, e.g., does not have a specific order or the like. Moreover, for example, the term "first component/member" does not imply the existence of a "second component/member", and the term "second component/member" does not imply the existence of the "first component/member". The terms "up", "down", "front/forward", "back/rear", "left", "right" and similar expressions used in the disclosure are for illustrative purposes only and are not limiting.

Embodiments of the present disclosure provide a surgical instrument driving and transmission mechanism and assembly mechanism for a surgical robot. Embodiments of the present disclosure are described below with reference to the accompanying drawings. In the present disclosure, the surgical instrument driving and transmission mechanism mainly includes three major parts: an instrument drive, a sterile adapter, and a surgical instrument.

The instrument drive is mounted on a sliding arm at an end of the surgical robot and can move up and down on the sliding arm. There are a plurality of driving motors in the instrument drive, for example, three driving motors, four driving motors, or five driving motors, etc. Each driving motor corresponds to an output shaft. A respective driving motor provides power to an effector of the surgical instrument through a respective output shaft and a respective drive transmission member connected with the respective output shaft. For example, the respective driving motor is connected with the output shaft, to transmit power to the effector of the surgical instrument through a transmission structure on a surface of the instrument drive, a transmission structure of the sterile adapter, and a transmission structure of the surgical instrument, so as to achieve pitch, yaw, and grip of a claw of the surgical instrument.

The sterile adapter avoids a direct contact between the instrument drive and the surgical instrument. The sterile adapter is connected on a sterile curtain. The sterile curtain and the sterile adapter wrap at least one robotic arm and the instrument drive of the surgical robot, so that the at least one robotic arm and the instrument drive are isolated from the outside. The sterile adapter is connected to an upper end surface of the instrument drive, and the sterile adapter is provided with adapter transmission members to transmit power.

The surgical instrument is meshed with the sterile adapter. The surgical instrument is provided with instrument transmission members. The instrument transmission members cooperate with the adapter transmission members of the sterile adapter, and then the surgical instrument is driven to achieve pitch, yaw, and grip of the surgical instrument by the instrument drive.

In the embodiment, in order to describe a positional relationship of various mechanism components, a plurality of directional terms are defined, and the direction terms are interpreted as follows. A longitudinal direction of a shaft of the surgical instrument is defined as a Z direction or a vertical direction, a span direction of two opposite sides of a surgical instrument box, the instrument drive, or the sterile adapter is defined as an X direction, and a direction from the back of the surgical instrument to the shaft of the surgical instrument is defined as a Y direction. The terms "up/upper" and "down/lower" indicate visual angles of the surgical instrument, the sterile adapter, and the instrument drive in a usage state. The terms "front" and "rear" are defined from the view of the shaft of the surgical instrument and the back of the surgical instrument box, for example, a direction towards the shaft of the surgical instrument is defined as "front", and a direction towards the back of the surgical instrument box is defined as "rear". "Left" and "Right" are directions (left and right directions) based on two opposite sides of the surgical instrument box from the perspective of looking directly into the shaft of the surgical instrument.

Specific embodiments of the present disclosure will be described below in conjunction with specific embodiments and the accompanying drawings.

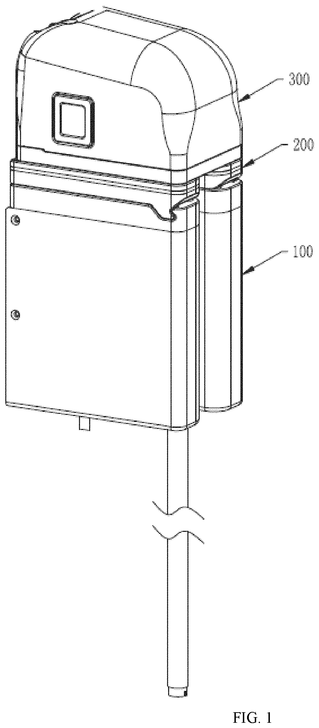

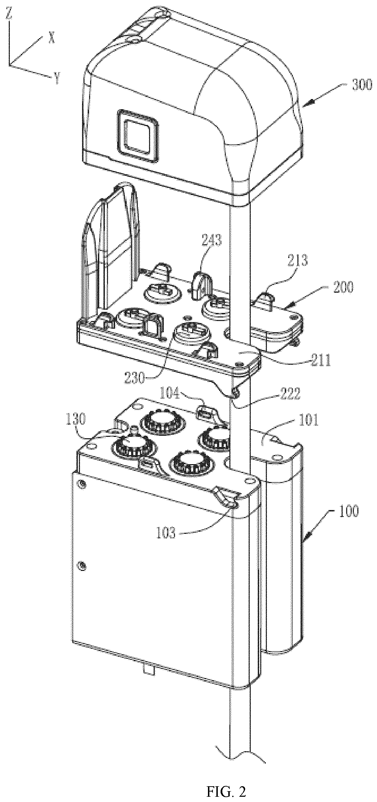

FIG. 1 is a schematic structural view of an instrument drive 100, a sterile adapter 200, and a surgical instrument 300 that are assembled. As shown in FIG. 2, the instrument drive 100, the sterile adapter 200, and the surgical instrument 300 each have an adapted configuration on their upper and/or lower surfaces (i.e., a X-Y surface), so that an upper surface of the instrument drive 100, an upper surface and a lower surface of the sterile adapter, and a lower surface of a surgical instrument box are not visible after they are assembled.

The following first describes connection between transmission portions of the instrument drive 100, the sterile adapter 200, and the surgical instrument 300 in detail.

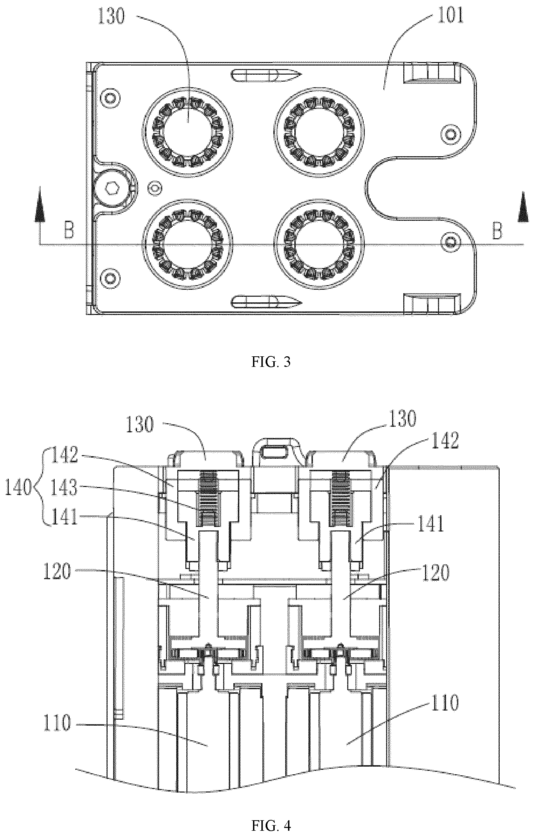

As shown in FIG. 2, the instrument drive 100 has a regular-shaped housing with one or more driving motors 110 inside. Each of the plurality of driving motors 110 is connected with a respective output shaft 120, so that the number of output components of the instrument drive 100 is the same as the number of driving motors. The instrument drive 100 has an upper surface and the upper surface is used as an assembly surface for being connected with an assembly surface of the sterile adapter 200. As shown in FIG. 3, the instrument drive defines one or more mounting holes on the assembly surface 101 of the instrument drive (mounting holes are shown in the figures, but are not labeled) for the output components of the instrument drive. In embodiments of the disclosure, the output components of the instrument drive 100 include one or more drive transmission members 130, such that the mounting holes defined on the assembly surface of the instrument drive are also used as mounting holes (not indicated in the drawings) for the drive transmission members. A respective drive transmission member 130 of the drive transmission members 130 is located in a respective mounting hole for the respective drive transmission member and an upper end of the respective drive transmission member 130 may protrude from the assembly surface 101 of the instrument drive.

As shown in FIGS. 3 and 4, the output components of the instrument drive further include a plurality of elastic assemblies 140. A respective elastic assembly 140 is located below the respective drive transmission member. The respective elastic assembly 140 includes a first spring seat 141 fixed to the respective output shaft, a second spring seat 142 fixed to the respective drive transmission member 130, and a spring 143 located between the first spring seat 141 and the respective drive transmission member 130. The first spring seat 141 and the second spring seat 142 are engaged to seal the spring in a cavity defined by the first spring seat 141, the second spring seat 142, and the respective drive transmission member 130. In a natural state, since the spring 143 can apply pushing force to the two spring seats on both sides of the spring 143, the first spring seat 141 and the second spring seat 142 can be engaged with each other at edge positions of the first spring seat 141 and the second spring seat 142, and the respective drive transmission member 130 can be lifted and protruded from the assembly surface 101 of the instrument drive.

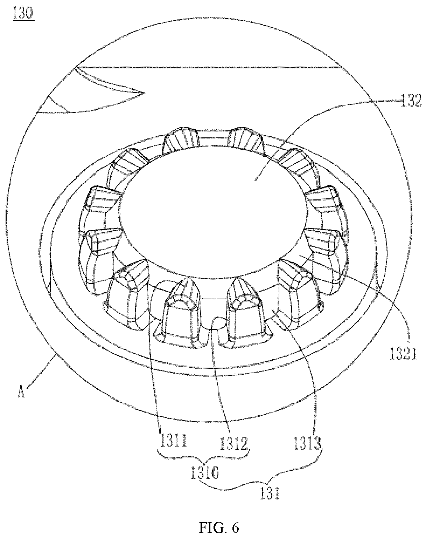

The respective drive transmission member 130 has a substantially cylindrical structure and a circular cross section. A rotation axis of the respective drive transmission member 130 is located at the center of the circular structure. As shown in FIG. 6, an upper end of the respective drive transmission member 130 is a closed structure and the respective drive transmission member 130 is provided with a first tooth-like portion 131 at an upper end or a top end of the closed structure. The first tooth-like portion includes a plurality of first teeth 1310 evenly spaced and arranged in a radial direction of the respective drive transmission member 130, i.e., the first teeth 1310 are arranged in a radial pattern. There may be odd numbers or even numbers of first teeth 1310. A top end, i.e., a tooth peak 1311, of a respective first tooth 1310 is optionally sharp or rounded, to avoid having a platform at the tooth peak as much as possible. The respective first tooth 1310 has two meshing surfaces 1312 on two opposite sides of a lower end of the respective first tooth 1310. A respective meshing surface 1312 may be a plane in a vertical direction or a slightly inclined surface with respect to the vertical direction. The respective first tooth 1310 is or is not symmetrical with respect to a centerline of the respective first tooth 1310, but in any case, the first tooth-like portion 131 is centrally symmetrical on the drive transmission member 130. Each two adjacent first teeth 1310 are spaced apart from each other by a respective first tooth slot 1313. The respective drive transmission member 130 is further provided with a structure for guiding and positioning (i.e., guide positioning structure), such as a disc surface portion 132, at the top of the respective drive transmission member. The disc surface portion 132 may be flush with or higher than (extend beyond) a plane where tooth peaks 1311 of the plurality of first teeth 1310 are located. The disc surface portion 132 may include a bevel portion 1321 provided in a circumferential direction of the disc surface portion 132 for guiding. In other embodiments, there is no disc surface portion at the top of the drive transmission member. Specifically, a cylinder structure is provided at an axle center of the drive transmission member, and a bevel guide structure is provided at the top of the cylinder in a circumferential direction of the cylinder using a same guiding principle, which are not shown in the figures.

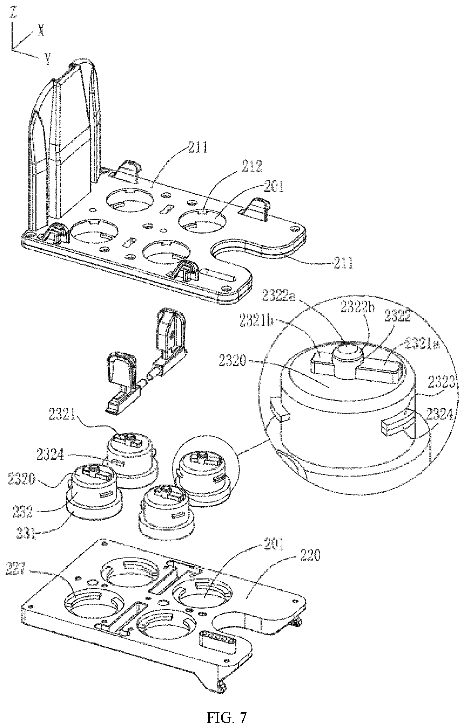

As shown in FIG. 7, the sterile adapter 200 mainly includes an upper housing 210, a lower housing 220, and one or more adapter transmission members 230. The lower housing 220 has a lower surface 221 capable of matching the assembly surface 101 of the instrument drive and the upper housing 210 has an upper surface capable of matching a lower surface 301 of the surgical instrument box 310. In a complete housing structure consisting of the upper housing 210 and the lower housing 220, one or more mounting holes 201 for the adapter transmission members are defined on the housing structure and penetrating the housing structure. A respective adapter transmission member 230 is located in a respective mounting hole 201 for the adapter transmission member, so that a lower end portion 231 of the respective adapter transmission member 230 can be cooperated with the upper end of the drive transmission member 130, and an upper end portion 232 of the adapter transmission member 230 can be cooperated with a lower end of a respective instrument transmission member 311.

The respective adapter transmission member 230 has a main body, and the main body has a cylindrical shape and has a circular or annular cross section. A rotation axis of the respective adapter transmission member 230 is located at the center of the circular structure. In other words, after the respective adapter transmission member is engaged with the respective drive transmission member, the respective adapter transmission member and the respective drive transmission member are coaxial. The sterile adapter may be a structure with one end closed, forming a partition between the drive transmission member and the instrument transmission member in space.

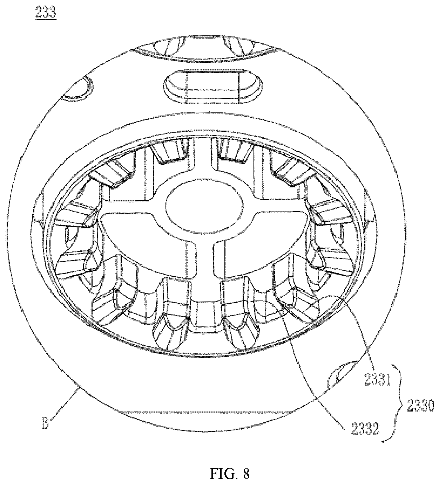

As shown in FIG. 8, the lower end portion 231 of the respective adapter transmission member 230 has a second tooth-like portion 233. Similar to the first tooth-like portion, the second tooth-like portion 233 includes a plurality of second teeth 2330 evenly spaced and arranged in a radial direction of the adapter, i.e., the second teeth 2330 are arranged in a radial pattern. A tooth peak 2331 of a respective second tooth 2330 may be either sharp or rounded. The respective second tooth 2330 has two meshing surfaces 2332 on two opposite sides of the respective second tooth 2330. A meshing surface 2332 on each of the two opposite sides of the respective second tooth 2330 can be meshed/engaged with a meshing surface 1312 of an adjacent first tooth 1310 facing the meshing surface 2332. That is, the respective second tooth 2330 can be inserted into the respective first tooth slot 1313 defined between two adjacent first teeth 1310, and the meshing surface of the respective second tooth is in clearance fit with the meshing surface of the adjacent first tooth. In the illustrated embodiment, the second tooth-like portion is located at an inner periphery of the main body of the adapter transmission member 230, the first tooth-like portion is located at an outer periphery of a main body of the drive transmission member 130, and a state in which the second tooth-like portion 233 is combined with the first tooth-like portion 131 is like that the second tooth-like portion forms an engaging cover on the first tooth-like portion. In some embodiments, the second tooth-like portion may be located at an outer periphery of the main body of the adapter transmission member 230 and the first tooth-like portion may be located at an inner periphery of the main body of the drive transmission member, such that a state in which the second tooth-like portion is combined with the first tooth-like portion is like that the second tooth-like portion is embedded in the first tooth-like portion. In other embodiments, the first tooth-like portion and the second tooth-like portion may have an identical shape, and both the tooth-like portions are exposed structures, such that a complete cylinder can be formed when the two are coupled. It shall be understood that the transmission structure with meshed tooth-like portions by adopting the same principle also falls within an equivalent/alternative scheme of this embodiment.

The upper end portion 232 of the respective adapter transmission member 230 is configured to be connected with the respective instrument transmission member. As shown in FIG. 7, the upper end portion of the respective adapter transmission member 230 has an end surface 2320, which can be firmly attached to an end surface of the instrument transmission member 3110. The respective adapter transmission member further includes a transmission boss 2321 protruding from the end surface of the respective adapter transmission member. In some embodiments, there may be one or more transmission bosses on the respective adapter transmission member, and the one or more transmission bosses are in an eccentric configuration on the adapter transmission member. That is, the one or more transmission bosses are in a non-axisymmetric distribution, or in a non-planar symmetric distribution, or in a non-centrosymmetric distribution. The transmission boss illustrated in the figures can be deemed as a relatively large transmission boss 2321, which crosses a central position of the respective adapter transmission member 230, but two ends of the transmission boss 2321 are not at a same distance from the central position. Alternatively, the transmission boss illustrated in the figures can be deemed as two transmission bosses: a transmission boss 2321a and a transmission boss 2321b, and the two transmission bosses 2321a and 2321b have different lengths/sizes. In the embodiments of the disclosure, the transmission bosses located at both ends of the central position of the respective adapter transmission member 230 can balance the transmission of torque as much as possible, and in addition, the asymmetric distribution of the transmission bosses can make the adapter transmission member and the instrument transmission member at a back end of the instrument only have a unique alignment position when they are engaged with each other.

The surgical instrument 300 includes a surgical instrument box 310 located at the back end of the surgical instrument 300, a surgical tool (not shown) located at a forward end of the surgical instrument 300, a shaft 320 connected between the surgical instrument box and the surgical tool, and a transmission mechanism such as a steel wire in the shaft 320.

As described above, the lower surface 301 of the surgical instrument box 310 is fitted with the upper surface 211 of the sterile adapter 200. The surgical instrument box has a mounting seat at the lower end of the surgical instrument box, and a cylinder structure is provided on the mounting seat for connecting the transmission mechanism, such as the steel wire, and there are one or more instrument transmission members 311 fixed on a lower end of the cylinder structure.

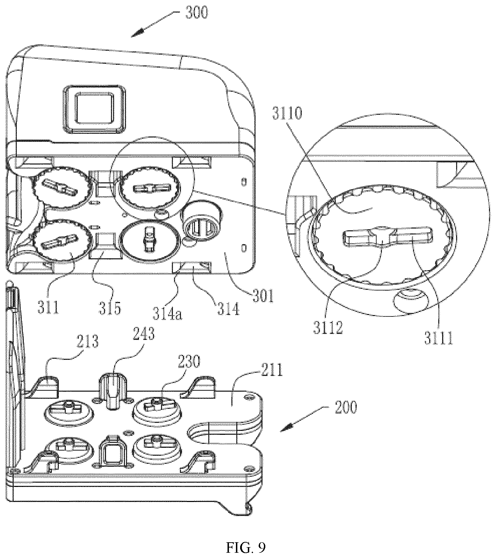

As shown in FIG. 9, a respective instrument transmission member is fitted with the respective adapter transmission member. The respective instrument transmission member includes at least one transmission sinking table 3111. A respective transmission sinking table 3111 is fitted with a respective transmission boss 2321. The respective transmission sinking table 3111 forms an inwardly recessed structure on the instrument transmission member 311, i.e., the respective transmission sinking table 3111 is recessed inwardly from an end surface of the instrument transmission member 311. The respective transmission sinking table 3111 is fitted to the respective transmission boss 2321 in shape, so that the respective transmission boss is in clearance fit with the respective transmission sinking table, or the respective transmission boss and the respective transmission sinking table are at least adapted in a direction of power transmission. For example, contact points and contact surfaces of torque formed in a rotation direction of the respective adapter transmission member 230 and the respective instrument transmission member 311 are adapted. There is no restriction on an inwardly recessed depth of the respective transmission sinking table 3111, as long as the respective transmission boss 2321 is ensured to fit with the respective transmission sinking table 3111.

In the embodiment, as shown in FIG. 7, the respective adapter transmission member 230 further includes a cylindrical boss 2322 protruding from the end surface 2320 of the respective adapter transmission member 230. The cylindrical boss 2322 is coaxial with the respective adapter transmission member 230. That is, the cylindrical boss is located at the central position of the respective adapter transmission member. In embodiments of the disclosure, a height of the cylindrical boss 2322 is higher than a height of the transmission boss 2321. That is, a distance between an end surface of the cylindrical boss and the end surface of the adapter transmission member is greater than a distance between an end surface of the transmission boss and the end surface of the adapter transmission member. Similarly, as shown in FIG. 9, the respective instrument transmission member 311 is further provided with a cylindrical sinking table 3112 fitting with the cylindrical boss 2322. The cylindrical sinking table 3112 is in clearance fit with the cylindrical boss. The cylindrical sinking table 3112 is recessed inwardly from the end surface 3110 of the respective instrument transmission member 311. In addition, a depth of the cylindrical sinking table 3122 ensures the insertion of the cylindrical boss without hindering fit between the transmission boss 2321 and the transmission sinking table 3111. Thus, the back end of the instrument and the cylindrical structure are pre-connected before the assembly of the transmission member at the back end of the instrument is completed.

In some embodiments, when the cylindrical boss and the transmission boss are physically connected, e.g., as shown in the figures, the cylindrical boss and the transmission boss are formed in an integral structure and have no gap therebetween. A diameter of the cylindrical boss should be greater than a width of the transmission boss and correspondingly, a diameter of the cylindrical sinking table for fitting with the cylindrical boss should be greater than a width of the transmission sinking table in the instrument transmission member at the back end of the instrument. For example, as illustrated in the figures, a width between two torque surfaces of the transmission boss 2321 is smaller than the diameter of the cylindrical boss 2322, so that the pre-connecting is effective.

In an embodiment, in order to calibrate an absolute position of the instrument, as shown in FIG. 7, the respective adapter transmission member of the sterile adapter further includes at least one positioning part 2323, and the at least one positioning part 2323 is located at an outer periphery of the respective adapter transmission member of the sterile adapter. At least one limiting part 212 is formed inside the respective mounting hole for the respective adapter transmission member of the sterile adapter, and each of the at least one limiting part 212 is substantially located at a position of the upper housing 210 of the sterile adapter near the upper surface 211. When the at least one limiting part 212 and the at least one positioning part 2323 are substantially at a same horizontal plane, the adapter transmission member of the sterile adapter rotates, so that a respective positioning part 232 rotates to a position at which a respective limiting part 212 is located, and the respective limiting part 212 prevents the respective positioning part 2323 from rotating, and in this case, the adapter transmission member of the sterile adapter cannot continue to rotate, and such a position can be used as an original position of the sterile adapter.

The respective adapter transmission member further includes at least one stop part 2324, and the at least one stop part 2324 is located at the outer periphery of the respective adapter transmission member. The respective adapter transmission member further includes at least one abutment protrusion 227 provided at the bottom of the respective mounting hole for the respective adapter transmission member and configured to prevent the at least one stop part 2324 from moving. Therefore, even if the sterile adapter is not mounted, since the structure of the sterile adapter is complete, the adapter transmission member is not easy to fall off.

In one embodiment, the respective positioning part is higher than the respective stop part on the adapter transmission member of the sterile adapter. The at least one limiting part 212 at the top of the respective mounting hole for the adapter transmission member prevents the at least one positioning part from rotating, and the at least one abutment protrusion 227 at the bottom of the respective mounting hole for the adapter transmission member prevents the at least one stop part 2324 from moving.

In one embodiment, the respective stop part 2324 and the respective positioning part 2323 may be formed in an integral structure.

The following describes connection between non-transmission portions of the instrument drive, the sterile adapter, and the surgical instrument.

The instrument drive and the sterile adapter are engaged with each other. The instrument drive and the sterile adapter are provided with two sets of engaging positions along two opposite sides of the instrument drive. Each set of engaging positions include at least two engaging positions. In one set of engaging positions, the assembly surface of the instrument drive is rigidly fitted with the assembly surface of the sterile adapter. That is, an upper cover of the instrument drive is integrally formed with an engaging surface and the lower housing of the sterile adapter is integrally formed with an engaging surface, and the two engaging surfaces are fitted with each other to form connection between the instrument drive and the sterile adapter. In the other set of snap-fit positions, the instrument drive and the sterile adapter are connected by means of operable engagement assemblies.

Specifically, the assembly surface 101 of the instrument drive and the assembly surface of the sterile adapter are fitted to each other in terms of size and shape. The instrument drive is provided with at least one hook-shaped recessed portion 103 each forming a recess, and a surface of a respective hook-shaped recessed portion 103 is integrally formed with the assembly surface 101 of the instrument drive. That is, a member constituting the surface of the respective hook-shaped recessed portion (i.e., a hook-shaped surface of the instrument drive) is integrally formed with an upper housing of the instrument drive. The respective hook-shaped recessed portion 103 is located at a position close to a front side of the instrument drive.

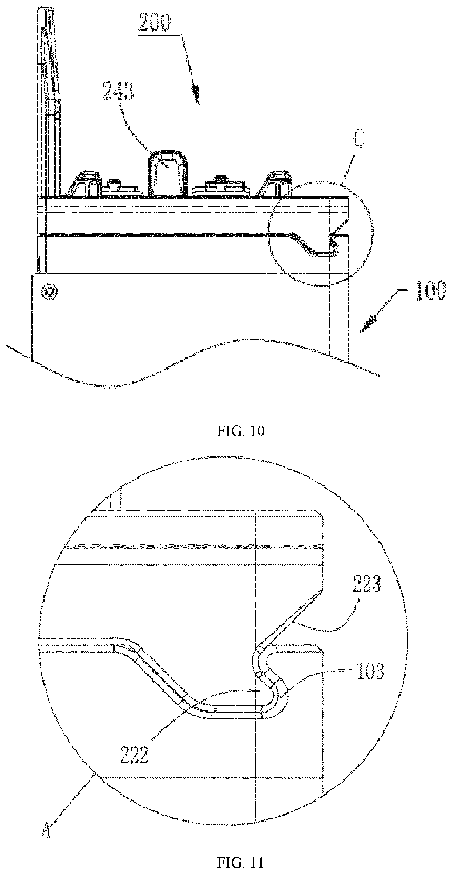

In an embodiment, as shown in FIGS. 10, 11, and 12, the respective hook-shaped recessed portion 103 has a width in a left-right span direction of the instrument drive, and the respective hook-shaped recessed portion includes a plurality of continuous curved surfaces, a plurality of continuous planar surfaces, or a combination of at least one of the plurality of curved surfaces and at least one of the plurality of planar surfaces from an end to another end of two opposite ends of the respective hook-shaped recessed portion in a front-rear direction of the instrument drive.

Accordingly, the sterile adapter has at least one hook-shaped member 222 that protrudes from the lower surface 221 of the sterile adapter. A surface of a respective hook-shaped member 222 is integrally formed with the lower surface 221 of the sterile adapter. That is, a member constituting the surface of the respective hook-shaped member 222 of the sterile adapter (i.e., a hook-shaped surface of the sterile adapter) is integrally formed with the lower housing of the sterile adapter. A respective hook-shaped member 222 of the at least one hook-shaped member 222 is located at a position close to the front side of the sterile adapter.

In an embodiment, there are two hook-shaped members 222, and the two hook-shaped members 222 are located on two opposite sides of the sterile adapter, respectively. The respective hook-shaped member 222 has a width, substantially the same as the width of the respective hook-shaped recessed portion, in a left-right span direction of the sterile adapter. The respective hook-shaped member includes a plurality of continuous curved surfaces, a plurality of continuous planar surfaces, or a combination of at least one of the plurality of continuous curved surfaces and at least one of the plurality of planar surfaces from an end to another end of two opposite ends of the respective hook-shaped member in a front-rear direction of the sterile adapter. The respective hook-shaped recessed portion and the respective hook-shaped member are visually complementary in shape.

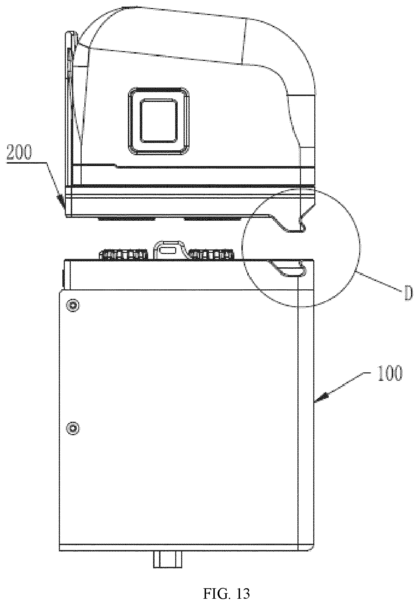

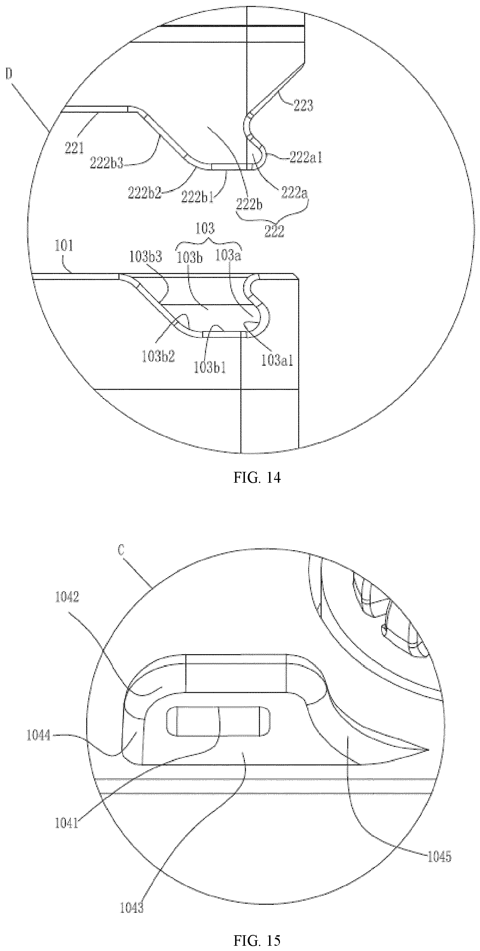

In embodiments of the disclosure, as illustrated in FIGS. 13 and 14, the respective hook-shaped recessed portion 103 mainly includes a front portion 103a close to the front side of the instrument drive and a rear portion 103b close to a rear side of the instrument drive. A structure above the front portion 103a is a solid structure of the upper housing of the instrument drive and the front portion 103a is a receiving space for receiving a front end of the respective hook-shaped member. The front portion 103a has a surface 103a1, which refers to a substantially "S"-shaped surface of the solid structure of the upper housing of the instrument drive in the left-right span direction of the instrument drive. The rear portion 103b has a surface including a flat surface 103b1 that connects with the surface 103a1 of the front portion, a flat arc surface 103b2, and an inclined surface 103b3 that connects with the assembly surface 101 of the instrument drive.

Accordingly, the respective hook-shaped member mainly includes a front portion 222a close to the front side of the sterile adapter and a rear portion 222b close to a rear side of the sterile adapter. The front portion 222a is a solid structure, and the solid structure has a surface 222a1, which is substantially in a shape of "S" in the left-right span direction of the sterile adapter. The rear portion 222b of the respective hook-shaped member is also a solid structure. The solid structure constituting the rear portion 222b has a flat surface 222b1 that connects with the surface 222a1, an arc surface 222b2, and an inclined surface 222b3 that connects with the lower surface 221 of the sterile adapter in the left-right span direction.

During installation, the respective hook-shaped member 222 of the sterile adapter is inserted into the respective hook-shaped recessed portion 103 of the instrument drive. The hook-shaped surface of the respective hook-shaped member 222 and the respective hook-shaped recessed portion 103 of the instrument drive are connected in a wedged manner and thus have a force component in the vertical direction. In addition, with aid of the hook-shaped recessed portion 103 and the hook-shaped member 222, the sterile adapter is cooperated with the instrument drive without use of external members. More importantly, the cooperation between the sterile adapter and the instrument drive with aid of the hook-shaped recessed portion 103 and the hook-shaped member 222 has a certain rigidity, which avoids higher accumulated tolerances caused by too many external members, movable members, elastic members, complex members, and the like to a certain extent.

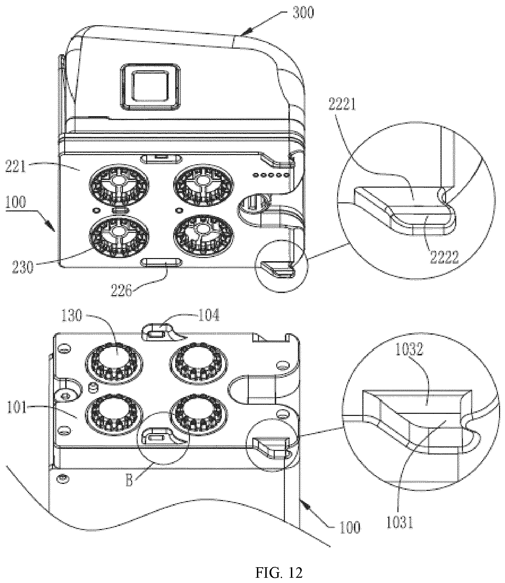

In an embodiment, as shown in FIG. 12, the respective hook-shaped recessed portion 103 of the instrument drive may have one or two straight side portions 1031, and each side portion 1031 may be a vertical surface structure or an inclined surface structure. There is a smooth arc-shaped structure or curved surface structure on part of an upper end of the respective side portion 1031, and the smooth arc-shaped structure or the curved surface structure can be regarded as a guide portion 1032 for the respective hook-shaped recessed portion. The respective hook-shaped member 222 of the sterile adapter may also have one or more straight side portions 2221. A respective side portion 2221 can fit with the respective side portion 1031 of the respective hook-shaped recessed portion, and the respective hook-shaped member may also have a smooth curved surface or arc-shaped surface structure at a lower end 2222 of a side surface of the respective hook-shaped member. Therefore, the respective side portion 1031 of the respective hook-shaped recessed portion can be firmly fitted with the respective side portion 2221 of the respective hook-shaped member, to limit movement of the hook-shaped member 222 in the left-right direction (i.e., X direction) when the hook-shaped member is inserted into the hook-shaped recessed portion, thereby limiting the movement of the sterile adapter 200 in the X direction. In addition, the guide portion 1032 of the hook-shaped recessed portion and the curved surface or arc-shaped surface structure at the lower end 2222 of the hook-shaped member have a guiding effect, which facilitates that the hook-shaped member of the sterile adapter is more smoothly and naturally inserted into the hook-shaped recessed portion of the instrument drive.

In an embodiment, as shown in FIG. 12, the sterile adapter further includes an avoidance portion 223 on the front side of the lower end of the sterile adapter, that is, in front of the upper end of the hook-shaped member. The avoidance portion 223 is a structure with a bevel surface or a smooth arc-shaped surface. The bevel surface or the smooth arc-shaped surface of the avoidance portion 223 smoothly transits with the hook-shaped surface of the hook-shaped member. That is, the hook-shaped surface of the hook-shaped member and the bevel surface roughly constitute a "<"-shaped structure when viewed from the left side. An upper wing of "<"-shaped structure is the bevel surface or the smooth arc-shaped surface of the avoidance portion. A lower wing of the "<"-shaped structure is a part of the hook-shaped surface of the hook-shaped member 222.

The fitting of the hook-shaped surface of the sterile adapter and the hook-shaped surface of the instrument drive can be regarded as a first connection manner between the sterile adapter and the instrument drive. In addition, there is a second connection manner between the sterile adapter and the instrument drive, and the second connection manner is achieved through connection of operable engagement assemblies. A position at which the second connection manner is achieved is spaced apart from a position at which the first connection manner is achieved. That is, the second connection manner and the first connection manner are achieved on different positions spaced on the fitting surfaces (assembly surfaces) of the sterile adapter and the instrument drive and having a spacing therebetween in the front-back direction, i.e., in the Y direction.

Specifically, as shown in FIGS. 12 and 15, the instrument drive is provided with at least one first fixing engaging member 104, and the sterile adapter further includes at least one first movable engaging member 241. A respective first fixing engaging member 104 and a respective first movable engaging member 241 may abut against each other in the vertical direction. That is, the respective first fixing engaging member 104 has a downward abutment surface 1041, and the respective movable engaging member 241 has an upward abutment surface 2411.

The at least one first fixing engaging member 104 protrudes from the upper surface 101 of the instrument drive and is integrally formed with the upper housing of the instrument drive. The respective first fixing engaging member 104 is in a shape of a projection, and defines an engaging space at a roughly intermediate position thereof. The engaging space can accommodate the respective first movable engaging member. The top of the engaging space is the abutment surface 1041 of the first fixing engaging member, so that the first movable engaging member and the first fixing engaging member are engaged with each other in the vertical direction.

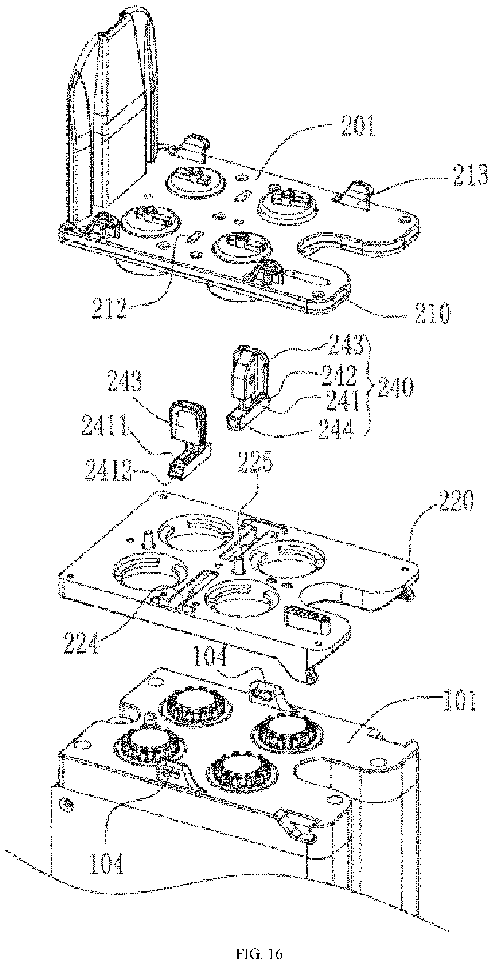

As shown in FIG. 16, the sterile adapter is provided with at least one first movable member 240. A respective first movable engaging member 241 is located at the bottom of a respective first movable member 240. The respective first movable member 240 further includes a first operating portion 242 located at the top of the first movable member 240 and a first operating button 243 connected with the first operating portion 242. The respective first movable member 240 further includes a guide portion 244. The sterile adapter defines a guide groove 224 for accommodating the guide portion, and the guide portion 244 can be inserted into the guide groove 224 and moved in an extension direction of the guide groove 224. The guide groove 224 is extended in the left-right span direction of the sterile adapter, that is, the X direction. Similarly, the guide portion 244 is moved along the left-right span direction of the sterile adapter. The guide portion 244 is positioned in the guide groove 224 and abuts against a spring 225 in the guide groove 224. The spring 225 provides a thrust force to drive the guide portion 244 or the first movable member 240 to move in the left-right direction of the sterile adapter, i.e., in a direction away from the transmission portion of the sterile adapter. In the embodiment, the sterile adapter has the upper housing 210 and the lower housing 220, and the guide groove 224 is defined on the lower housing 220. Both ends of the guide groove 224 are closed, so that the guide portion 244 can merely move in the guide groove 224. The upper housing of the sterile adapter defines an opening 214 substantially above the guide groove 224, and the opening 214 is used for providing a moving space for the operating portion. Due to the driving force of the spring, a locking position of the movable member 240 is a position at which the movable member abuts against the guide groove and is away from the transmission portion of the sterile adapter, and an unlocking position of the movable member is a position at which the movable member is close to transmission portion of the sterile adapter by manually moving the operating portion. In the illustrated embodiment, there is no linkage between two movable members of the sterile adapter, and the two movable members are respectively connected with springs. During operation, operating buttons of the two movable members are pinched and squeezed to make the two movable members move towards a direction close to each other, that is, towards a direction close to the transmission portion of the sterile adapter, so as to complete the movement of the movable engaging members from the locking position to the unlocking position. In some embodiments, the guide grooves for accommodating the two guide portions may be connected with each other, and a spring is connected between the two guide portions, which may cause the linkage of the two guide portions or the two movable members.

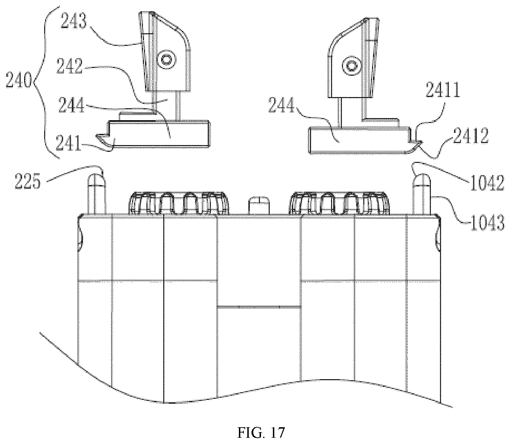

In the embodiment, as shown in FIG. 17, the lower end of the respective first movable engaging member 241 has an oblique wedge surface 2412, that is, there is a sloping upward plane located directly below the abutment surface 2411 of the respective movable engaging member. During installation, the oblique wedge surface 2412 of the first movable engaging member 241 and the top end of the first fixing engaging member 104 can be squeezed only by pressing the sterile adapter downward without pinching tightly the first operating button 243, such that the first movable member 241 can be moved from the locking position to the unlocking position.

In an embodiment, as shown in FIGS. 9 and 16, the first operating portion 242 of the respective first movable member 240 protrudes from the upper surface 211 of the sterile adapter, i.e., the first operating button 243 is located above the upper surface 211 of the sterile adapter. The surgical instrument box is provided with at least one groove 315 for the at least one first operating button 243. A respective groove 315 is used for accommodating a respective first operating button 243. The first operating portion 242 and the first operating button 243 are hidden by the surgical instrument box after assembly. In some embodiments, the first operating portions of the first movable members may be disposed on both sides of the sterile adapter, such that the operating portions or operating buttons of the sterile adapter are exposed.

The first fixing engaging member 104 of the instrument drive is also fitted with the lower surface of the sterile adapter in a specific configuration, so that the movable engaging part not only has the function of engaging, but also has the function of guiding and tightly fitting. Specifically, as shown in FIG. 15, the first fixing engaging member 104 of the instrument drive has a smooth curved-surface structure 1042 on each of all sides of front and rear sides and left and right sides at an upper end of the first fixing engaging member 104, and has a fitting surface 1043 that is straight in the vertical direction on each of both sides of the first fixing engaging member. In addition, the first fixing engaging member 104 has a smooth arc surface 1044 at a lower end of the first fixing engaging member 104 and close to the rear end of the sterile adapter, and the smooth arc surface 1044 extends in a Z-axis direction and around the Z-axis direction. Since connection process of the sterile adapter and the instrument drive is performed from the front end of the instrument drive to the rear end of the instrument drive, the first fixing engaging member 104 has a slope surface 1045 at a front end of the first fixing engaging member 104 for guiding, and the rear end of the first fixing engaging member 104 has the arc surface in the Z-axis direction.

Accordingly, as shown in FIG. 12, the lower housing 220 of the sterile adapter is provided with at least one recessed structure 226. A respective recessed structure 226 allows the respective first fixing engaging member 104 to be inserted into, and the respective first movable engaging member 241 is located in the respective first recessed structure 226. The respective recessed structure 226 has a smooth curved-surface structure in each of the X direction and the Y direction, and thus can be perfectly engaged with the respective first fixing engaging member 104.

Similar to the hook-shaped fitting surfaces, in the second connection manner, engaging of the fixing engaging member and the recessed structure of the sterile adapter for embedding of the fixing engaging member has a certain rigidity, which reduces the accumulated tolerance caused by movable connection of the movable engaging member and the fixing engaging member to a certain extent.

When the sterile adapter and the instrument drive are installed, the hook-shaped surface of the sterile adapter can be obliquely inserted into the hook-shaped recessed portion of the instrument drive, and then the rear end of the sterile adapter is pressed down to make the fixing engaging member of the instrument drive inserted into the recessed structure of the sterile adapter. Thereafter, the component force in the X direction is generated by squeezing the slop surface at the upper end of the fixing engaging member of the instrument drive with the sloping surface at the lower end of the movable engaging member of the sterile adapter, so that the movable member of the sterile adapter can squeeze the spring as a whole and move towards a direction close to the transmission portions of the sterile adapter. The sterile adapter drives the movable member to continue to press down, and the first movable member continue to be pressed down until the abutment surface of the first movable engaging member 241 is lower than the abutment surface with the first fixing engaging member, and the first movable engaging member 241 is inserted into the first fixing engaging member 104 by the push force of the spring to achieve engaging between the first movable engaging member 241 and the first fixing engaging member 104.

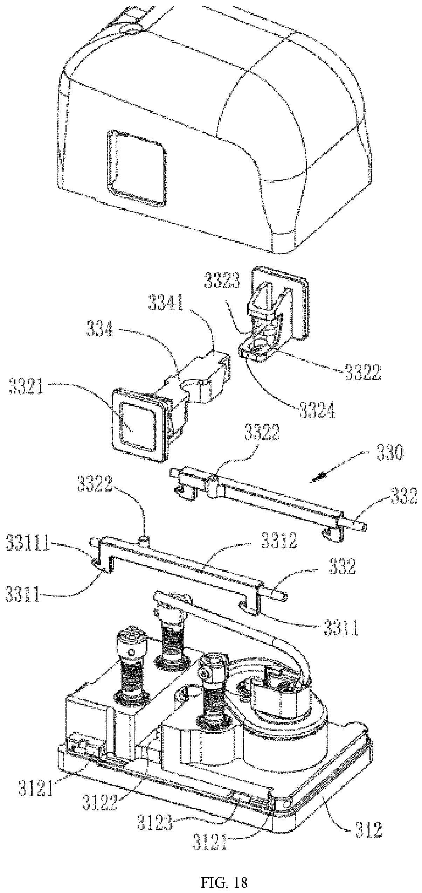

The connection between the sterile adapter and the surgical instrument is achieved through engaging of movable engaging members. Specifically, as shown in FIG. 9, the sterile adapter is provided with two sets of second fixing engaging members 213 on the upper surface 211 of the sterile adapter, that is, the two sets of second fixing engaging members 213 are integrally formed with the upper housing 210 of the sterile adapter. As shown in FIG. 18, the surgical instrument box 310 includes a seat body 312 for mounting a steel wire rope column, and the instrument transmission member of the surgical instrument is coupled to a lower end of the steel wire rope column. There is at least one second movable engaging assembly 330 provided on the seat body 312. A respective second movable engaging assembly 330 includes at least one second movable engaging member 3311, and a respective second movable engaging member 3311 can be engaged with the respective second fixing engaging member 213 in the vertical direction.

As shown in FIG. 18, the respective second movable engaging assembly 330 includes at least one second movable member 331, and a respective second movable member 331 includes two second movable engaging members 3311 and a connecting arm 3312 located between the two second movable engaging members 3311. The connecting arm 3312 has a length, such that the two second movable engaging members 3311 are spaced apart from each other. The at least one second movable engaging assembly 330 is configured as two second movable engaging assemblies 330, connecting arms 3312 of the two second movable engaging assemblies 331 are respectively arranged on the left and right sides of the seat body, i.e., on the left and right sides of the surgical instrument box. The connecting arm 3312 is extended along the front-rear direction of the surgical instrument box, i.e., the Y direction. The connecting arm 3312 is fixedly connected to the two second movable engaging members 3311, so that the three are formed in an integral structure.

Due to restriction of the transmission portions in the surgical instrument box, a locking position and an unlocking position of the respective second movable engaging member 3311 are both limited to be located in a longitudinal direction of the respective connecting arm 3312, i.e., a moving direction of the respective second movable engaging member 3311 is along the longitudinal direction of the connecting arm 3312. Specifically, the respective second movable engaging assembly further includes a guide rod 332, and two opposite ends of the guide rod are fixedly connected with the seat body 312 through two guide rod seats 3121. The respective connecting arm 3312 defines a guide groove for the guide rod to pass through (the guide groove is shown in the figures, but is not labeled), and the guide rod 332 passes through the guide groove defined on the connecting arm, so that the connecting arm 3312 can only move along the guide rod. A distance between the two opposite ends of the guide rod 332 is a maximum distance that the connecting arm 3312 can move. A spring 333 is sleeved at an end of the guide rod 332, so that one end of two opposite ends of the spring 333 abuts against the connecting arm 3312 and the other end of the two opposite ends of the spring 333 abuts against the guide rod seat 3121. In a natural state, the connecting arm 3312 and the second movable engaging member 3311 can be driven to be at a locking position due to a driving force of the spring. Accordingly, an opening direction of an abutment surface of the respective second fixing engaging member 213 on the upper surface of the sterile adapter should be directed toward the moving direction of the respective second movable engaging member 3311. In embodiments of the disclosure, the guide rod 3312 and the connecting arm 3312 are located in the surgical instrument box, so that at least one hole 3123 is defined on the seat body 312, and a respective hole 3123 provides a space for the respective second movable engaging member 3311 to move and provides a position where the respective second movable engaging member 3311 can be engaged with the respective second fixing engaging member 213.

The respective second movable engaging assembly 330 further includes a second operating portion 332. In the embodiment, a moving direction of the second operating portion 332 is different from the moving direction of the connecting arm 3312. That is, the second operating portion 332 is not fixedly connected to the second movable member 331. Specifically, the second movable member 331 has a passive portion 3313 fixed to the connecting arm 3312. In some embodiments, the passive portion 3313 and other components of the second movable member 331 are integrally formed. The second operating portion 332 includes an actuating portion 3322 having an actuating surface, such that the passive portion 3313 and the actuating surface can form a line contact or a surface contact, and the actuating surface of the actuating portion 3322 can limit a moving path of the passive portion 3313. In some embodiments, the actuating surface may be a bevel surface or an arc-shaped surface and the passive portion may have a bevel surface or an arc-shaped surface. In some embodiments, the passive portion 3313 may also be provided as a shaft and the passive portion may form rolling on the actuating surface of the actuating portion.

In the embodiment, the second operating portion 332 further includes a second operating button 3321, and the second operating button 3321 is fixedly connected with the actuating portion 3322. A plurality of second operating buttons 3321 are arranged on both sides of the surgical instrument box. When a plurality of second operating portions 332 are used, the plurality of second operating portions 332 need to be pressed inwardly from both sides of the surgical instrument box, so that a moving direction of a respective second operating portion 332 is along a span direction of the surgical instrument box 310, that is, the X direction. The oblique actuating surface 3321 enables the force exerted on the passive portion 3313 to be decomposed into the force in the front-rear direction of the surgical instrument box 310, that is, the force in the Y direction, so that the actuating portion 3322 can drive the connecting arm and the second movable engaging members to move towards the unlocking position.

In an embodiment, the respective second movable assembly 330 further includes a guide member 334 that assists in guiding the movement of the second operating portion. Specifically, the second operating portion 332 further includes a second guide portion 3323. The guide member 334 and the second guide portion 3323 are engaged with each other through a plurality of surfaces of the guide member 334 and a plurality of surfaces of the second guide portion 3323 in the span direction of the surgical instrument box. That is, a guide direction of the guide member and the second guide portion is the X direction. A spring (not shown) is connected between the second guide portion and the guide member, such that one end of the spring abuts against the second operating portion 332 and the other end of the spring abuts against the guide member 334.

In the embodiment, the second operating portion 332 further defines a limiting hole 3324, and at least one limiting column 3122 is fixedly provided on the seat body 312. The limiting column passes through the limiting hole. Since the limiting column passes through the limiting hole, the second operating portion does not detach from the surgical instrument box, and both ends of the limiting hole become an unlocking position and locking position of the second operating portion.

Before the surgical instrument and the sterile adapter are engaged, it is necessary to guide the installation of the surgical instrument and the sterile adapter. The following describes an installation guide structure for the surgical instrument and the sterile adapter in detail.

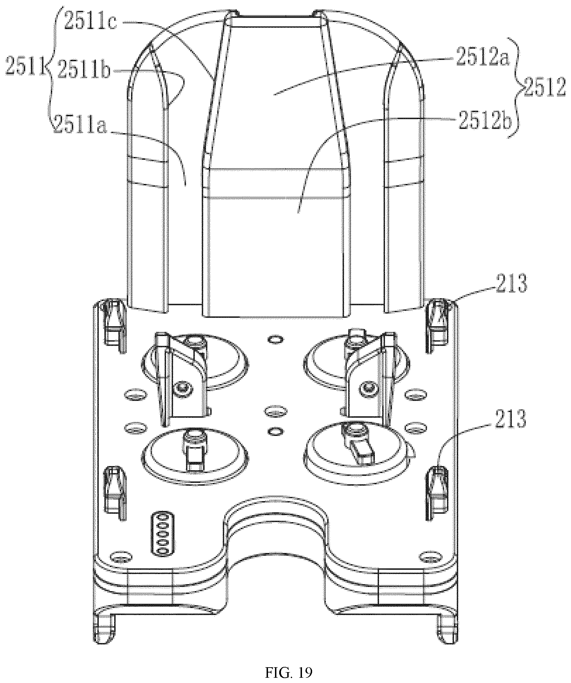

Specifically, as shown in FIG. 19, the sterile adapter 200 includes a backboard 250. The backboard is positioned at a rear side of a body structure 210 of the sterile adapter and perpendicular to the upper surface of the upper housing 210 of the sterile adapter. The backboard 250 has a side surface facing the surgical instrument, i.e., a front side surface 250a of the backboard. The front side surface is connected with a rear portion of the surgical instrument box 310. The backboard has a rear side surface 252b located at the rear of the backboard. The rear side surface 252b can be bonded to a sterile cloth along edges of the backboard to separate the sterile cloth from the surgical instrument. Alternatively, the rear side surface 252b can also exist separately, and the sterile cloth is bonded to the body structure of the sterile adapter.

The backboard 250 can isolate the surgical instrument 300 from the sterile cloth so as to prevent the sterile cloth from being damaged by friction when the sterile cloth is in contact with the surgical instrument 300. In addition, the backboard 250 can function as guiding and limiting during installation.

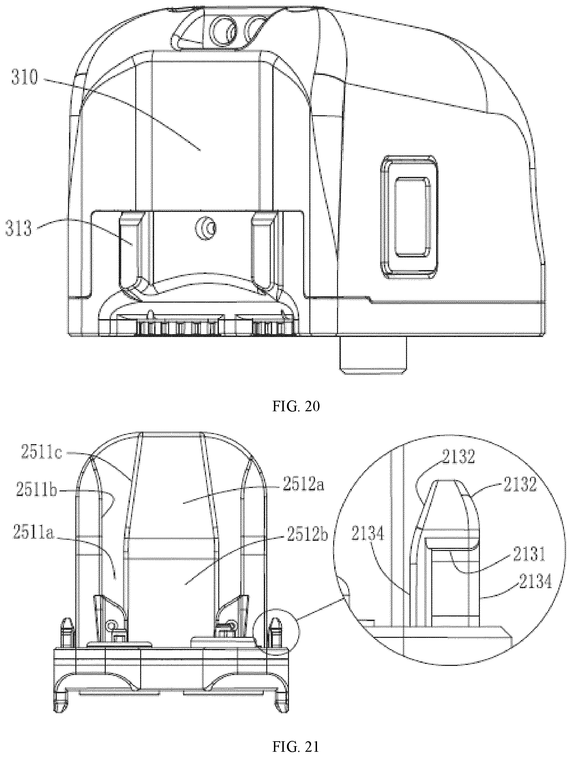

Specifically, the backboard 250 has a plate-like structure. At least one guide groove 2511 is defined on the front side surface 251a of the backboard 250 and runs through the backboard 250 from top to bottom in the vertical direction. As shown in FIG. 20, the surgical instrument box 310 is provided with at least one projection 312. A respective projection 312 is engaged with a respective guide groove 2511, i.e., the respective projection 312 is not wider than the respective guide groove 2511. The respective guide groove 2511 runs through the backboard 250 in the vertical direction, so that the surgical instrument can be mounted from top to bottom.

The respective guide groove 2511 has a plane 2511a perpendicular to the upper surface of the sterile adapter. The plane 2511a is located in an X-Z plane. The X-Z plane prevents the protrusion 312 from moving in the Y direction. When the surgical instrument is installed, the protrusion directly abuts against part of the X-Z plane, such that there is no need to adjust an installation position in the Y direction.

The respective guide groove 2511 further includes two walls 2511b and 2511c perpendicular to the X-Y plane. A distance between the two walls 2511b and 2511c in part of the guide groove 2511 varies from top to bottom along the Z direction, e.g., a width of the part of the guide groove is gradually decreased from top to bottom in the Z direction, that is, the part of the guide groove gradually changes from wide to narrow. The part of the guide groove can guide the protrusion to move in the X direction, that is, there is a certain space in the X direction for the protrusion to adjust a position of the protrusion. Moreover, when the projection 312 is initially inserted into the guide groove 2511, no additional alignment is required. Meanwhile, a distance between the two walls 2511b and 2511c in another part of the guide groove 2511 does not change, i.e., a width of the other part of the guide groove is constant. The width of the guide groove 2511 is gradually reduced until the guide groove fully fit with the protrusion, that is, the guide groove limits movement of the protrusion in the X direction, and the protrusion continues to move downwardly in the Z direction.

In an embodiment, the at least one guide groove is configured as one guide groove or more than one guide groove. For example, when two guide grooves are defined, a surface structure 2512 is provided between the two guide grooves. In order to enable the surgical instrument to be finely adjusted in the Y direction, an upper portion 2512a of the surface structure 2512 has an inclined surface to guide the surgical instrument to move in the Y direction. A lower portion 2512b of the surface structure 2512 has a vertical surface, that is, a surface parallel to the X-Z surface, which limits movement of the surgical instrument in the Y direction.

The above is the preliminary guidance and positioning for connection between the surgical instrument and the sterile adapter. In embodiments, connection between the surgical instrument box and the sterile adapter can also be achieved through precise guidance and precise positioning. In an embodiment, a precise guidance and positioning structure is provided in combination with a connecting structure used in the non-transmission portions of the sterile adapter and the surgical instrument box.

Specifically, the sterile adapter is provided with two sets of second fixing engaging members 213 on the upper surface of the sterile adapter. In the embodiment, the two sets of second fixing engaging members 213 are integrally formed with the upper housing 210 of the sterile adapter. A respective second fixing engaging member 213 of the sterile adapter has an abutment surface 2131, and the abutment surface 2131 of the respective second fixing engaging member is facing downward, so that an upper end of the second fixing engaging member does not need to be connected with the movable engaging member of the surgical instrument box.

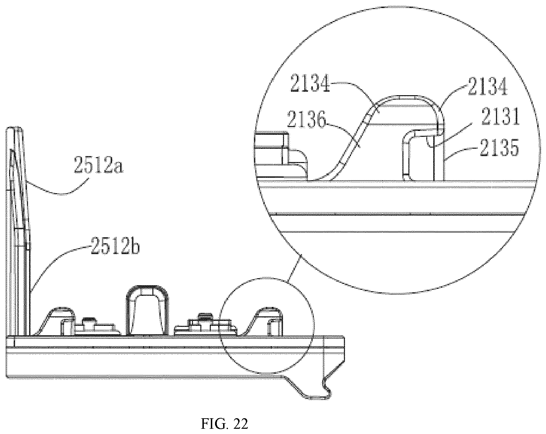

More specifically, as shown in FIGS. 21 and 22, the second fixing engaging member 213 has smooth curved-surface structures at the upper end of the second fixing engaging member 213 in both the X direction and the Y direction. In the embodiment, the upper end of the second fixing engaging member 213 has first guide surfaces 2132 respectively extending towards the left and right sides of the second fixing engaging member. The upper end of the second fixing engaging member has second guide surfaces 2133 respectively extending toward the front and rear sides of the second fixing engaging member.

In one embodiment, there are fitting portions 2134, that are straight in the Y-Z plane, respectively on the left and right sides of the second fixing engaging member 213.

In one embodiment, there is an arc-shaped structure, that is extended around the Z-axis direction, at a lower end of a front side of the respective second fixing engaging member, i.e., at a position of the respective second fixing engaging member and near the front end of the sterile adapter, and the arc-shaped structure is a vertical positioning part 2135 of the respective second fixing engaging member.

In one embodiment, there is a slope structure at a lower end of a rear side of the respective second fixing engaging member, that is, at a position located on the second fixing engaging member and close to the backboard, and the slope structure is a third guide portion 2136 of the second fixing engaging member.

In one embodiment, the first guide surfaces, the second guide surfaces, the straight fitting portions, the vertical positioning part, and the third guide portion of the second fixing engaging member are all in continuous smooth transition.

Accordingly, as shown in FIG. 9, the surgical instrument box defines at least one second recess 314 for the at least one second fixing engaging member 213 on the lower surface 301 of the surgical instrument box and configured to allow the at least one second fixing engaging member to be embedded into. The respective second movable engaging member of the surgical instrument is located within a respective second recess 314. The respective second recess has smooth curved-surface structures in X direction and Y direction, and can be perfectly fitted with the respective second fixing engaging member.

In the embodiment, the respective second recess 314 has smooth curved-surface structures at an entrance 314a of the respective second recess 314 in both the X direction and the Y direction, and there is a curved surface, that is fitted with the upper end of the respective second fixing engaging member 213, inside the recess.

The second fixing engaging member of the sterile adapter and the recessed structure of the surgical instrument in the embodiment can not only be used for the connection of the sterile adapter and the surgical instrument, but also play a guiding role. In addition, due to the fitting of the surfaces of the sterile adapter and the surgical instrument, the fitting surface has a certain degree of rigidity, which reduces the accumulated tolerance caused by movable engaging of the movable engaging member and the fixing engaging member to a certain extent.

In the present embodiment, in order to facilitate the description of the installation of the instrument drive, the sterile adapter, and the surgical instrument, the assembly of the transmission portions and the assembly of the non-transmission portions are respectively described. However, in actual operation, the assembly process is a complete and continuous process in which the sterile adapter is first installed on the instrument drive, and then the surgical instrument is installed on the sterile adapter. The instrument drive, the sterile adapter, and the surgical instrument each are a complete structure. During installation, the instrument drive, the sterile adapter, and the surgical instrument can be assembled as follows.

In the embodiment, the installation process of the sterile adapter and the instrument drive is as follows. The front end of the sterile adapter is tilted downward, the hook-shaped member is inserted into the hook-shaped recessed portion of the instrument drive, and the guide portion of the hook-shaped recessed portion and the lower end of the hook-shaped member function as guiding to adjust the front end of the sterile adapter in the left-right direction, i.e., perform the X-direction micro-movement. The rear end of the sterile adapter is pressed downward, and the curved-surface structures on the left and right sides of the respective fixing engaging member of the instrument drive cooperate with the recessed structure of the sterile adapter, forming a micro-adjustment of the rear end of the sterile adapter in the X-direction, until the side portion located in the hook-shaped recessed portion is attached to the side portion of the hook-shaped member and the fitting surfaces on the left and right sides of the fixing engaging member are attached to the vertical surfaces of the recessed structure. In this case, the side portion of the hook-shaped recessed portion and the side portion of the hook-shaped member, and the fitting surfaces on the right and left sides of the fixing engaging member form a limitation in the X-direction, so that a position of the sterile adapter is no longer adjusted in the X direction. The rear end of the sterile adapter continues to be pressed downwardly, and the curved-surface structure at the rear end of the first fixing engaging member of the instrument drive cooperates with the recessed structure of the sterile adapter to form a Y-direction guide, which is capable of adjusting the slight movement of the sterile adapter in the Y-direction. In addition, due to the guiding effect of the recessed structure of the sterile adapter and the fixing engaging member, the hook-shaped member at the front end of the sterile adapter continues to be pushed forward, and the rear end of the sterile adapter is further pressed down, such that the lower end of the first movable engaging member of the sterile adapter abuts against the upper end of the first fixing engaging member of the instrument drive. Due to the action of the beveled surface at the lower end of the first movable engaging member and the beveled surface at the upper end of the first fixing engaging member, the first movable member is caused to move toward the transmission portion of the sterile adapter, and then the rear end of the sterile adapter continues to be pressed down, such that when the abutment surface of the first movable engaging member moves to the abutment surface of the first fixing engaging member, the first movable engaging member is reset due to the driving force of the spring. Moreover, in this case, the hook-shaped member at the front end of the sterile adapter is pushed forward until the hook-shaped member is completely inserted into the hook-shaped recessed portion, and the hook-shaped surface of the hook-shaped member is completely fitted with the hook-shaped surface of the hook-shaped recessed portion. Therefore, the Z-axis surface located at the rear side of the fixing engaging member is completely cooperated with the recessed structure of the sterile adapter. In this process, the transmission members of the sterile adapter and the transmission members of the instrument drive are mounted in place in any direction by the tooth-like portions, and the output shaft of the instrument drive lifts the drive transmission members of the instrument drive and the adapter transmission members of the sterile adapter, so that the limiting part of the sterile adapter and the positioning part on the sterile adapter are on a substantially horizontal level, and then the motor of the instrument drive rotates to drive the drive transmission member of the instrument drive and the adapter transmission member of the sterile adapter to rotate, so that the positioning part abuts against the limiting part. In this case, the initial position of the adapter transmission member of the sterile adapter can be calibrated.

The installation process of the surgical instrument and the sterile adapter is as follows. The installation starts from the back end of the surgical instrument, the surgical instrument is moved above the sterile adapter, and the protrusion of the surgical instrument abuts against the plane of the guide groove of the backboard of the sterile adapter. Thereafter, the surgical instrument moves downwardly along the guide groove, until the upper end of the guide groove completes rough guidance for the surgical instrument. The protrusion on the surgical instrument box makes the rear end of the surgical instrument no longer swing left and right. The surgical instrument is roughly positioned by the lower end of the guide groove of the backboard of the sterile adapter, and then the surgical instrument continues to move downwardly, the entrance of the recess of the surgical instrument box is in contact with the upper end of the fixing engaging member of the sterile adapter, and then the surgical instrument box is guided by the fitting of each surface of the recess and the fixing engaging member to complete accurate guidance, so that the surgical instrument box and the sterile adapter are accurately aligned. Thereafter, the surgical instrument box continues to move downwardly, so that the movable member and the fixing engaging member are engaged. In this case, the instrument transmission member of the surgical instrument is aligned with the adapter transmission member of the sterile adapter, and the cylindrical structure of the adapter transmission member of the sterile adapter is inserted into the cylindrical sinking table of the instrument transmission member of the surgical instrument to form pre-connecting. The transmission boss on the adapter transmission member of the sterile adapter is not aligned with the transmission sinking table of the instrument transmission member of the surgical instrument, causing the transmission boss on the adapter transmission member of the sterile adapter to abut against the surface where the instrument transmission member of the surgical instrument is located, and the transmission boss on the adapter transmission member of the sterile adapter is pressed downwardly, causing the positioning part to detach from the limiting part. Thereafter, the motor of the instrument drive rotates to drive the adapter transmission member of the sterile adapter to rotate until the transmission boss on the adapter transmission member of the sterile adapter and the transmission sinking table of the instrument transmission member of the surgical instrument are aligned and combined to complete the connecting and installation. In this case, an angle of rotation of the adapter transmission member of the sterile adapter relative to an initial position is an absolute position of the surgical instrument, and the absolute position can be used to determine various angles of action of the surgical instrument.

Unless otherwise defined, technical and scientific terms used herein have the same meanings as are commonly understood by those skilled in the art of the present disclosure. Terms used herein are for specific practical purposes only and are not intended to limit the present disclosure. Terms such as "disposed/arranging" that appear herein may denote either a part is attached directly to another part or a part is attached to another part through a middle part. Features described herein in one embodiment may be applied to another embodiment alone or in combination with other features unless the features are not applicable in the other embodiment or otherwise noted.

The present disclosure has been described by way of the embodiments described above, which are for the purpose of example and illustration only and are not intended to limit the disclosure to the scope of the described embodiments. Further variations and modifications may be made in accordance with the teachings of the present disclosure which fall within the scope of the claims of the present disclosure.

## Claims

1. An instrument driving and transmission mechanism for a surgical robot, comprising:
an instrument drive including a drive transmission member exposed at an upper surface of the instrument drive;
a sterile adapter, wherein the sterile adapter has a lower surface matching the upper surface of the instrument drive, the sterile adapter includes an adapter transmission member penetrating a body of the sterile adapter, and the adapter transmission member has a lower end configured to cooperate with the drive transmission member; and
a surgical instrument, wherein the surgical instrument has a back end matching an upper surface of the sterile adapter, the surgical instrument includes an instrument transmission member exposed at the back end of the surgical instrument, and the instrument transmission member is configured to cooperate with an upper end of the adapter transmission member;
wherein the adapter transmission member has a surface on which a plurality of teeth are provided and evenly spaced, the drive transmission member has a surface on which a plurality of teeth are provided and evenly spaced, the plurality of teeth of the adapter transmission member are configured to be meshed with the plurality of teeth of the drive transmission member, and the adapter transmission member and the instrument transmission member are configured to cooperate with each other by means of transmission bosses.

2. The instrument driving and transmission mechanism of claim 1, wherein the adapter transmission member includes a first tooth-like portion at the lower end of the adapter transmission member, the first tooth-like portion includes the plurality of teeth evenly spaced, and the plurality of teeth on the first tooth-like portion are arranged in a radial pattern; and
wherein the instrument drive includes a second tooth-like portion at an upper end of the instrument drive, the second tooth-like portion includes a plurality of teeth evenly spaced, and the plurality of teeth on the second tooth-like portion are arranged in a radial pattern.

3. The instrument driving and transmission mechanism of claim 2, wherein a respective tooth of the plurality of teeth of the first tooth-like portion has a sharp or rounded tooth peak, and/or a respective tooth of the plurality of teeth of the second tooth-like portion has a sharp or rounded tooth peak.

4. The instrument driving and transmission mechanism of claim 2 or 3, wherein a respective tooth of the plurality of teeth of the first tooth-like portion has meshing surfaces on two opposite sides of a tooth peak of the respective tooth, a respective tooth of the plurality of teeth of the second tooth-like portion has meshing surfaces on two opposite sides of a tooth peak of the respective tooth, and the meshing surfaces of the respective tooth of the first tooth-like portion are in clearance fit with the meshing surfaces of the respective tooth of the second tooth-like portion.

5. The instrument driving and transmission mechanism of any of claims 1 to 4, wherein the adapter transmission member includes at least one transmission boss protruding from an upper surface of the adapter transmission member, and the at least one transmission boss is in an eccentric distribution with respect to a rotation axle center of the adapter transmission member;
wherein the instrument transmission member includes at least one transmission sinking table on a surface of the instrument transmission member, and the at least one transmission sinking table is in an eccentric distribution with respect to a rotation axle center of the instrument transmission member, wherein a respective transmission boss of the at least one transmission boss is in clearance fit with a respective transmission sinking table of the at least one transmission sinking table.

6. The instrument driving and transmission mechanism of claim 5, wherein the adapter transmission member further includes a cylindrical boss protruding from the upper surface of the adapter transmission member, the cylindrical boss has a height larger than a height of the respective transmission boss, and the cylindrical boss is coaxial with the adapter transmission member; and
wherein the instrument transmission member further includes a cylindrical sinking table on the surface of the instrument transmission member, the cylindrical sinking table is coaxial with the instrument transmission member, and the cylindrical boss is in clearance fit with the cylindrical sinking table.

7. The instrument driving and transmission mechanism of claim 6, wherein the at least one transmission boss is configured as two transmission bosses, the two transmission bosses and the cylindrical boss are formed in an integral structure, a diameter of the cylindrical boss is larger than a width of the respective transmission boss, and a cylindrical structure between two transmission sinking tables on the instrument transmission member is configured to be fitted to the cylindrical boss.

8. The instrument driving and transmission mechanism of any of claims 1 to 7, wherein the sterile adapter includes an adapter body, the adapter body defines a mounting hole for the adapter transmission member, and the adapter transmission member is rotatably received in the mounting hole, wherein the adapter body is provided with a limiting part in the mounting hole, the adapter transmission member is further provided with a positioning part at an upper portion of the adapter transmission member, and the limiting part prevents the positioning part from moving horizontally.

9. The instrument driving and transmission mechanism of any of claims 1 to 8, wherein the adapter body further comprises a backboard perpendicular to the upper surface of the sterile adapter, and the backboard defines a guide groove on a surface of the backboard facing the surgical instrument, wherein the guide groove runs through the backboard from top to bottom, the guide groove has an upper portion and a lower portion, and the upper portion has a width larger than a width of the lower portion, wherein the surgical instrument is provided with a protrusion on a surface of the surgical instrument matching the backboard, and the protrusion is configured to be fitted to the lower portion of the guide groove.

10. The instrument driving and transmission mechanism of claim 9, wherein fitting surfaces of the sterile adapter and the surgical instrument each further comprise a fitting positioning surface.

11. The instrument driving and transmission mechanism of claim 10, wherein the sterile adapter is further provided with a fixing engaging member protruding from the upper surface of the sterile adapter, and the fixing engaging member has a plurality of guide surfaces from top to bottom, wherein the surgical instrument further has a recess for the fixing engaging member on a lower surface of the surgical instrument, and the recess has a shape configured to be fitted to a shape of the fixing engaging member.

12. An instrument driving and assembly mechanism for a surgical robot, comprising:
an instrument drive having an assembly surface connected with a sterile adapter;
the sterile adapter, wherein the sterile adapter has a lower surface matching the assembly surface of the instrument drive; and
a surgical instrument box, wherein the surgical instrument box has a lower surface matching an upper surface of the sterile adapter;
wherein the lower surface of the sterile adapter is configured to cooperate with the assembly surface of the instrument drive through hook-shaped surfaces and operable engagement assemblies, and the upper surface of the sterile adapter is connected with the lower surface of the surgical instrument box through at least two operable engagement assemblies spaced apart from each other.

13. The instrument driving and assembly mechanism of claim 12, wherein the instrument drive is provided with at least one fixing engaging member on the assembly surface of the instrument drive, and a respective fixing engaging member of the at least one fixing engaging member has a guide fitting surface and an abutment surface; and
wherein the sterile adapter includes at least one movable engaging member, and a respective movable engaging member of the at least one movable engaging member is configured to be engaged with the respective fixing engaging member, and wherein the sterile adapter further includes at least one recessed structure on the lower surface of the sterile adapter, and a respective recessed structure of the at least one recessed structure is configured to be fitted to the guide fitting surface of the respective fixing engaging member.

14. The instrument driving and assembly mechanism of claim 12 or 13, wherein the sterile adapter is provided with a hook-shaped member protruding from the lower surface of the sterile adapter, and the instrument drive is provided with a hook-shaped recessed portion, on the assembly surface of the instrument drive, for matching the hook-shaped member of the sterile adapter.

15. The instrument driving and assembly mechanism of any of claims 12 to 14, wherein the sterile adapter is further provided with at least one operating portion each at a connecting position of a respective operable engagement assembly through which the sterile adapter is connected with the instrument drive, the at least one operating portion protrudes from the upper surface of the sterile adapter, and the surgical instrument has a space for accommodating the at least one operating portion.
